# EUROPEAN PATENT APPLICATION

(11) **EP 1 258 730 A1**
(43) Date of publication of application: **20.11.2002**
(21) Application number: 01904323.1
(22) Date of filing: 07.02.2001
(51) Int. Cl.: G01N 33/543, G01N 33/566

(54) **METHOD FOR DETECTING SUBSTANCE**

(30) Priority: 07.02.2000 JP 2000029830
(71) Applicant: KYOWA MEDEX CO., LTD., Tokyo 104-0042 (JP)
(72) Inventor: MIIKE,A. Kyowa Medex Res.Lab. Kyowa Medex Co.Ltd., Nagaizumi-cho Sunto-gun, Shizuoka 4 (JP); MORI,H. Kyowa Medex Res.Lab. Kyowa Medex Co.Ltd., Nagaizumi-cho, Sunto-gun Shizuoka (JP)
(74) Representative: VOSSIUS & PARTNER
(86) International application number: JP0100833
(87) International publication number: WO01059454

(57) **Abstract**

The present invention relates to a method of detecting a substance to be detected in a sample characterized by mixing a sample containing the substance to be detected, in a solution, with carriers which have a shape such that, when suspended in a solution and flowed through a hollow tube for detecting signals formed in a signal detecting means capable of detecting a signal A and a signal B, two or more of the carriers do not move in an overlapping manner to the cross section of the hollow tube, to which a substance capable of binding to the substance to be detected (hereinafter referred simply to as "binding element") is bound through or not through a spacer, and which have an identifiable signal A; binding the substance to be detected to said binding element thereby causing a signal B to be formed on the carriers; flowing a suspension of said carriers through the hollow tube by a solution supply means connected to the hollow tube; detecting the signal A and the signal B by the signal detecting means; and relating the signal A with the signal B.

SEQUENCE LISTING
<110> KYOWA MEDEX CO., LTD

<120> Method of detecting substance

<130> 11275W01

<140>
<141>

<150> JP 2000-029830
<151> 2000-02-07

<160> 7

<170> PatentIn Ver. 2.0

<210> 1
<211> 15
<212> DNA
<213> Artificial Sequence

<220>
<223> Description of Artificial Sequence: Synthetic DNA

<400> 1
aaattgagta gttgc                                                    15

<210> 2
<211> 15
<212> DNA
<213> Artificial Sequence

<220>
<223> Description of Artificial Sequence: Synthetic DNA

<400> 2
aaaaacatct ccggg                                                    15

<210> 3

<211> 14
<212> DNA
<213> Artificial Sequence

<220>
<223> Description of Artificial Sequence: Synthetic DNA

<400> 3
ggtcaggaac aaaa                                                    14

<210> 4
<211> 24
<212> DNA
<213> Artificial Sequence

<220>
<223> Description of Artificial Sequence: Synthetic DNA

<400> 4
ttgttcctga ccgcaactac tcaa                                         24

<210> 5
<211> 24
<212> DNA
<213> Artificial Sequence

<220>
<223> Description of Artificial Sequence: Synthetic DNA

<400> 5
tacgatatgg ctccttggaa ctcg                                         24

<210> 6
<211> 24
<212> peptide
<213> Artificial Sequence

<220>
<223> Description of Artificial Sequence: Synthetic peptide

<400> 6
Ile Ser Phe Glu Asn Thr Trp Leu Trp His Pro Gln Phe Ser Ser
1               5                   10

<210> 7
<211> 24
<212> peptide
<213> Artificial Sequence

<220>
<223> Description of Artificial Sequence: Synthetic peptide

<400> 7
Ile Ser Phe Glu Asn Thr Trp Leu Trp Thr Ser Pro Phe Ser Ser
1               5                   10

## Description

### Technical Field

The present invention relates to a method of detecting a specific substance, a reagent for detecting a specific substance and an apparatus for detecting a specific substance.

### Background Art

In recent years, genetic information on humans, animals, plants and microorganisms has been analyzed and novel genes have been found one after another. However, the functions of proteins produced by such novel genes have not been elucidated almost at all and a long period of time and a long work will be required before the functions are ascertained. Thus, development of a method for efficiently analyzing the functions of proteins is a very urgent problem.

To confirm the function of a protein, first, a substance having an affinity to the protein is searched for and then the expression of the protein is genetically controlled utilizing organisms thereby to presume the role of the protein. In so doing, it is important to detect an affinity between the protein and the substance. One of such methods comprises packing a protein to be purified in a column after being immobilized on a carrier, passing various substances that are presumed to have an affinity with the protein through the column, and detecting whether the substance passes the column or adsorb thereto.

In recent years, chips on which proteins are immobilized have been developed as an efficient means [BioEssays, 21, 781 (1999)]. The method using a chip comprises binding a specific protein on a specific site of a chip using a semiconductor technique, contacting a sample with the chip to bind a substance to the protein and detecting such binding by a tag. This method uses a semiconductor chip, and always requires very small processing. Also, detection was impossible when the proteins immobilized on a chip were mutually bound through something or other. Furthermore, once a chip is prepared, it is impossible to readily add other kinds of proteins thereto, so a chip must be prepared again.

Genetic mutation is currently being examined and utilized for the diagnosis of diseases, effect of pharmaceuticals, and suppression of their side effects. However, as genetic mutation is widely variable, it is necessary to have a variety of DNA sequences available and detect DNA that hybridizes with each gene. Although a DNA chip has been used to solve this problem, it involved the same problems as those mentioned above.

Also known is a method using a so-called beads array which comprises the steps of; impregnating beads having a diameter of several µm with 2 to 3 kinds of fluorescent dyes at various ratios; analyzing the fluorescent light emitted by the resulting beads at two wavelengths; by using the ratio of the fluorescence intensities at the two wavelengths as a means to identify individual beads, acting a substance to be detected such as a protein, a DNA, etc. thereon; after binding or hybridization, trapping the beads in very small pockets; and carrying out analysis using fluorescence intensity [US Patent No. 5,843,767, Analytical Chemistry, 70(7), 1242(1998)]. However, this method involves various problems. For example, it requires a highly skilled technique for the measurement as it uses a special device having small holes. Also, because identification is made by delicate differences in the ratio of fluorescence intensities at two wavelengths, the discrimination sensitivity is low and it is not possible to deal with large varieties of proteins or of tens and thousands of DNA. Furthermore, in case dusts coming from samples or falling down from the surrounding environment contaminate when beads are taken in small microwells for detection, they will be noise components. In addition, such a method has a drawback that detection is not possible when proteins mutually have an affinity through or directly, not through, something.

Accordingly, development of a method of detecting a specific substance at a low cost with wide use has been desired.

### Disclosure of the Invention

An object of the present invention is to provide a method of detecting a specific substance at a low cost with wide use, a reagent for detecting a specific substance, and an apparatus for detecting a specific substance.

The present inventors found that a specific substance to be detected such as a variety of proteins, DNA, etc. can be detected without using expensive materials such as protein chips and DNA chips by: attaching to small carriers a signal A which is readable from outside; preparing various kinds of carriers in which a substance capable of binding to a substance to be detected such as proteins, DNA, etc. (hereinafter referred to as "binding element") is bound to the resulting carriers; binding the carriers to a substance in a test sample by mixing them, to form a new signal B; detecting the signals A and B almost simultaneously using a relatively simple apparatus; and carrying out analysis by contrasting the results of detection for the signal A and the signal B. Thus, the present invention has been completed.

That is to say, according to the present invention, through the use of carriers to which a signal A is attached and an apparatus for detection, results similar to those given by a protein chip or a DNA chip can be obtained in the absence of advanced techniques of the protein chip or the DNA chip by binding a substance capable of binding to a substance to be detected to the carriers. The present invention has characteristics that when various substances, each completely different, are to be detected, a number of carriers carrying a signal corresponding to the kind of the substances can be easily prepared and that a person conducting experiments can freely decide the combination of a signal and a sequence. In the case of a protein chip or a DNA chip, it is necessary to newly prepare a protein or DNA chip whenever the kind of a sample is changed, and therefore, wide applicability is lacking. In contrast, the carriers of the present invention can be prepared by binding a desired substance such as proteins, DNA, etc. to carriers to which a substance such as proteins, DNA, etc. has not been bound in accordance with conventional methods and can simply deal with a variety of substances at a low cost.

The present invention relates to the following (1)-(36).
(1) A method of detecting a substance to be detected in a sample characterized by mixing a sample containing the substance to be detected, in a solution, with carriers which have a shape such that, when suspended in a solution and flowed through a hollow tube for detecting signals formed in a signal detecting means capable of detecting a signal A and a signal B, two or more of the carriers do not move in an overlapping manner to the cross section of the hollow tube, to which a substance capable of binding to the substance to be detected (hereinafter referred simply to as "binding element") is bound through or not through a spacer, and which have an identifiable signal A; binding the substance to be detected to said binding element thereby causing a signal B to be formed on the carriers; flowing a suspension of said carriers through the hollow tube by a solution supply means connected to the hollow tube; detecting the signal A and the signal B by the signal detecting means; and relating the signal A with the signal B.
(2) A method according to the above (1), wherein said substance to be detected is a substance having a specific nucleotide sequence and said binding element is a substance having a nucleotide sequence complimentary to the nucleotide sequence of the substance to be detected or a partial sequence thereof.
(3) A method according to the above (2), wherein the binding of the substance to be detected to the binding element is carried out by hybridization of the complementary nucleotide sequences.
(4) A method according to the above (3), wherein the identifiable signal A is related with the nucleotide sequence of the binding element.
(5) A method according to the above (3), wherein the carriers have a mutually identifiable signal A which is related with the nucleotide sequence of the binding element and are in the form of a mixture of two or more of carriers having a mutually different signal A.
(6) A method according to any one of the above (2) to (5), wherein the binding element is DNA, RNA or PNA having 5 to 500 bases.
(7) A method according to any one of the above (3) to (6), wherein a signal B is formed on the carriers by binding a compound having a tag capable of recognizing hybridization between the nucleotide sequence of the binding element and the nucleotide sequence of the substance to be detected.
(8) A method according to the above (7), wherein said compound having a tag capable of recognizing the hybridization is a compound in which a compound having a nucleotide sequence complementary to the residual nucleotide sequence other than the sequence complementary to the nucleotide sequence of the binding element possessed by the substance to be detected is bound to a labeling compound.
(9) A method according to the above (8), wherein said compound having a nucleotide sequence complementary to the residual nucleotide sequence other than the sequence complementary to the nucleotide sequence of the binding element possessed by the substance to be detected is DNA, RNA or PNA having 5 to 500 bases.
(10) A method according to the above (7), wherein said compound having a tag capable of recognizing the hybridization is a compound in which an antibody recognizing the hybridization is bound to a labeling compound.
(11) A method according to the above (7), wherein said compound having a tag capable of recognizing the hybridization is a compound in which an intercalator recognizing the hybridization is bound to a labeling compound.
(12) A method according to the above (11) which further includes a step of separating the compound having a tag which has not been bound to the carriers from the carriers.
(13) A method according to any one of the above (7) to (12), wherein the labeling compound is a fluorescent substance, a light emitting substance, an enzyme or a compound which emits a fluorescent light or a luminescent light by means of an enzyme.
(14) A method according to the above (13), wherein the carriers are bound to a substance emitting a fluorescent light and the labeling compound is a compound which emits a fluorescent light by energy transfer.
(15) A method according to the above (7), wherein said compound having a tag capable of recognizing the hybridization is a compound which forms a signal B by intercalation.
(16) A method according to the above (1), wherein said binding element is a protein capable of binding to a substance to be detected.
(17) A method according to any one of the above (1) to (16), wherein the identifiable signal A is a digital signal.
(18) A method according to the above (17), wherein the digital signal A is magnetic or optical.
(19) A method according to the above (17), wherein the digital signal A is a bar code.
(20) A method according to any one of the above (1) to (19), wherein the shape of the hollow tube is cylindrical and the carrier is cylindrically shaped with its outer shape smaller than the inner diameter of the hollow tube, the length of said cylindrical shape being larger than the inner diameter of the hollow tube.
(21) A method according to any one of the above (1) to (20), wherein the carrier is cylindrical or spherical.
(22) A method according to the above (21), wherein the carrier internally contains magnetic particles.
(23) A method according to any one of the above (1) to (22), wherein the carrier is cylindrical and has an outer diameter of 1 µm to 2 mm and a length of 10 µm to 2 cm.
(24) A method according to any one of the above (1) to (23), wherein the carrier has a specific gravity of 0.8 to 2.0.
(25) A method according to any of the above (1) to (24), wherein the surface of the carrier is coated with a resin.
(26) A carrier to which a binding element capable of binding to a substance to be detected is bound through or not through a spacer and which has an identifiable signal A.
(27) A carrier according to the above (26), wherein said binding element has a nucleotide sequence complementary to the nucleotide sequence of a substance to be detected or a partial sequence thereof.
(28) A carrier according to the above (26), wherein said binding element is a protein capable of binding to a substance to be detected.
(29) A carrier according to any one of the above (26) to (28), which internally contains magnetic particles.
(30) A carrier according to any one of the above (26) to (29), wherein the signal A is a digital signal.
(31) A carrier according to the above (30), wherein said digital signal is a bar code.
(32) A carrier according to any one of the above (26) to (31), which is cylindrical or spherical.
(33) A carrier according to any one of the above (26) to (32), which is cylindrical and has an outer diameter of 1 µm to 2 mm and a length of 10 µm to 2 cm.
(34) A carrier according to any one of the above (26) to
(33) having a specific gravity of 0.8 to 2.0.
(35) A carrier according to any one of the above (26) to
(34) having a surface coated with a resin.
(36) An apparatus to detect a specific substance,
characterized by having a signal detecting means, a data input means, a data processing means, and a data output means: said signal detecting means being provided with signal detectors A and B capable of detecting at least two kinds of signals A and B present on a carrier to which a binding element having a nucleotide sequence complementary to a nucleotide sequence to be detected or a partial sequence thereof is bound through or not through a spacer, said signal detectors A and B having a hollow tube through which said carrier is flowed, and said carrier having a structure such that the carriers do not move in an overlapping manner to the cross section of said hollow tube; and said data processing means being provided with a function to relate a signal A on the carrier with a specific substance to be detected using data and signals A and B, said data specifying said specific substance that has been related with the signal A on the carrier and being input from the data input means, and said signals A and B having been detected by the signal detecting means, and to output said related data to the data output means.

The present invention is described in detail below.

According to the present invention, carriers which have a shape such that, when suspended in a solution and flowed through a hollow tube for detecting a signal formed in a signal detecting means capable of detecting a signal A and a signal B, two or more of the carriers do not move in an overlapping manner to the cross section of the hollow tube, to which a substance capable of binding to a substance to be detected (binding element) bound through or not through a spacer, and which have an identifiable signal A are used.

The carriers to be used in the present invention are insoluble in a solution.

Any signals that are identifiable by sorting may be used for the signal A on the carrier, and digital signals are preferably used. As digital signals, there is no limitation so long as they are digitalized signals. For example, bar codes, fine dots, and signals expressed by a combination of a presence and an absence of very thin lines are mentioned, and bar codes are preferably used.

Methods for attaching a signal A to carriers are not limited and include, for example, a method using a printing machine which can print very thin bar codes, fine dots, very thin lines, etc.; a method to convert the reflectance or refractive index of the surface into a shape of lines and dots by using very thin laser beams; a method which comprises optically demagnifying a pattern consisting of a bar code, lines, and dots to minuteness using a lens, etc., changing the property of the surface of irradiated areas and then bar-coding a carrier by a chemical treatment or a physical treatment such as getting a toner adsorbed thereon, etc.; a method which comprises coating the surface of a carrier with a photosensitive polymer which hardens or degrades by light and which has been treated to contain a dye, a pigment, a carbon, etc., followed by irradiation of a light in the form of expressing a signal, and then providing a signal thereto by etching; a method of using a carrier entirely consisting of a photosensitive resin; and a method to have a carrier internally contain a magnetic carrier in which a signal is recorded by an electromagnetic recording method.

The carrier may be of any shape so long as it enables reading of a digital signal attached thereto from outside. It is particularly preferable that the carrier has a shape such that carriers do not move in an overlapping manner to the cross section of a hollow tube when they are flowed through the tube. Also, a small carrier is preferable in view of the operability and the large surface area per volume. Particularly, for use in the detection method using a hollow tube to be mentioned below, the shape of the carrier may, for example, be spherical, polyangular prism-shaped and cylindrical though it depends upon the shape of the hollow tube, and cylindrical shape is preferable. A carrier of spherical shape can be used, for example, by attaching two or more of signals A such as two dimensional bar codes to the surface of the spherical carrier to make signal A readable from any positions, or by getting the carrier to internally contain magnetic particles and stopping the movement of the carrier by magnetic force for a short time when detection is carried out, thereby to face a specific side of the sphere toward the direction of detection by magnetic force.

As to the size of the carrier, in the case of a cylindrical carrier, for example, the diameter is preferably around 1 µm to 2 mm and the length is preferably around 10 µm to 2 cm. In case the carrier is spherical, a diameter of around 1 µm to 2 mm is preferred.

The specific gravity of the carrier is preferably around 0.8 to 2, more preferably 0.85 to 1.5, and most preferably 0.9 to 1.1. The difference in the specific gravity between a carrier-suspending solution and the carrier is preferably 0.1 or less, more preferably 0.05 or less, and most preferably 0.03 or less.

Shaping of the carrier may be carried out according to a method of molding a polymer into a polygon or a cylindrical shape, a method of cutting polygonal or cylindrical fibers by various means, etc. It is also possible to prepare a granular carrier according to the suspension polymerization method, and the emulsification polymerization method using a high molecular monomer, etc. In case the carrier is hollowcylindrical, such a shape can be formed by laminating a film made of a material that thermally shrinks in one direction and a film made of a material that undergo only a little shrinkage if any, attaching a signal A thereto, and heating the resulting film (with or without cutting) as heating causes shrinking of one layer only.

Examples of materials that undergo thermal shrinkage include finely cut or fibrous materials made of TFE shrinking tube, TOF shrinking tube, Teflon shrinking tube, FEP shrinking tube, etc. listed in Yamato Rika K.K.'s 2000-nen Plastic Kagakukiki Sogo Catalog, Plastic-hen (2000 Union Catalogs of Plastic Scientific Instruments; The Plastic Volume), nylon shrinkable tube, etc.

Particularly, it is preferable that the carrier or its surface consists of a substance which at least can not readily adsorb substances to be detected such as proteins, chemicals having a nucleotide sequence, etc. Although hydrophilic carriers are preferred, hydrophobic carriers can also be used because it is possible to reduce the adsorption on the surface of the carriers by finally treating the surface physically or with coating. Examples of natural polymers that may be used for coating include chitosan, proteins, hyaluronic acid, etc.

Although there is no particular restriction on the materials for the carrier, synthetic compounds are preferred. For example, polyacrylic acid or its esters, polymethacrylic acid or its esters, polyethers, polycarbonates, polyesters, poliamides, polyurethanes, polyimides, polystyrenes, polybutadien and polychloroprene, and copolymers and mixtures thereof are mentioned. Synthetic polymers generally used as raw materials for plastics and fibers are preferably used due to their low cost.

As synthetic polymer compounds used for the carrier, those containing, for example, a hydroxyl group, an ester group, a carboxyl group, an amide group, an amino group, an imino group, a hydroxysuccinyl ester group, a maleimide group, a glycidyl group, etc. in their side chains are preferable. Examples of monomers constituting these synthetic polymer compounds include ethyleneoxide-containing (metha)acrylic esters such as acrylic anhydride, methyl acrylate, ethyl acrylate, methyl methacrylate, ethyl methacrylate (these are hydrolyzed after polymerization), hydroxystyrene, ethylene glycol (metha)acrylate, triethylene glycol (metha)acrylate, etc., hydroxyl group-containing (metha)acrylic esters such as hydroxymethyl (metha)acrylate, hydroxypropyl (metha)acrylate, etc., epoxy group-containing (metha)acrylic esters such as glycidyl (metha)acrylate, etc., alkyl (metha)acrylic esters such as methyl (metha)acrylate, ethyl (metha) acrylate, etc., and monoethylene compounds such as acrylamide, methacrylamide, diacetoacrylamide, N-hydroxymethyl acrylamide, ethylenimine, acrylic hydroxysuccinylester, etc.

As the synthetic polymer compounds, homopolymers consisting of one kind of such monomers, and copolymers consisting of two or more mononers may be mentioned. These synthetic polymer compounds are required to form at least the surface layer of the carrier, and the internal part may consist of, for example, hydrophobic polymers such as polystyrene, polyacrylic ester, polyamide, polyvinyl, etc. It is preferable that the surface of the carrier, to which a binding element is bound through or not through a spacer, is prepared to contain a functional group. Polymer compounds in which these polymers have been made into hydrophilic by reaction are also used. Examples of suitable polymer compounds include polymers of glycidyl methacrylate.

The polymer compounds can be synthesized according to known methods, though the method to be used depends upon the kinds of monomers and polymer compounds.

A spacer is a stuff which binds the carrier to a binding element. Examples of the spacers include polyethylene glycol diglycidylether chains, single-strand DNA chains, single-strand RNA chains, single-strand peptide nucleic acid (abbreviated to PNA) and peptide chains. As the length of a polyethylene glycol diglycidylether chain, a length of 1 to 10 ethylene units is preferable, and of 1 to 5 ethylene units is more preferable. In the case of using a single-strand DNA chain and a single-strand RNA chain as a spacer, the chains may have any nucleotide sequences, and those having adenine (A), guanine (G), and cytosine (C) at the terminuses, and containing an amino group in the base are preferred. It is preferable that these single-strand DNA chains and single-strand RNA chains are of 3 to 40 mer in length, more preferably 5 to 20 mer in length. Peptides with chains of 1 to 50 mer are preferable, and 2 to 30 mer more preferable.

Any binding elements that are capable of binding to a substance to be detected may be used without restriction. For example, substances having a nucleotide sequence complementary to the nucleotide sequence possessed by a substance to be detected in a sample or a partial sequence thereof, and proteins and peptides capable of binding to a substance to be detected may be mentioned.

Any substances having a nucleotide sequence may be subjected to detection. As nucleotide sequences or their partial sequences to be detected, those derived from genes participating in various characters, genes participating in diseases, etc. are preferred. Examples of genes include HCV gene, cancer suppressor genes, WT-1, Klotho gene, GyrB gene, drug resistance genes, and obesity gene.

As proteins capable of binding to a substance to be detected, various receptors, antibodies against various antigens, lectin, etc. may be mentioned. As binding elements, ligands or antigens which bind to the receptors or antibodies can be used. Examples of receptors include VEGF receptor, IL-2 receptor, Fc receptor, etc. Furthermore, examples of antibodies include anti-HCV antibody, anti-HBs antibody, anti-HBe antibody, anti-GAD antibody, anti-CRP antibody, anti-CEA antibody, anti-C peptide antibody, anti-insulin antibody, anti-BNP antibody, anti-troponin-T antibody, anti-oxidized LDL antibody, anti-TBGL antibody, anti-MPB64 antibody, and anti-telomerase antibody, etc.

When the binding element is a protein, proteins purified from an extract of tissues or cells, or from a cell in which the gene of a desired protein is introduced thereto and expressed can be used as a binding element as such or after introducing a functional group thereto. In case the binding element is a low molecular compound, such compounds can be used as they are as a binding element if they have a functional group. If they do not have a functional group, they can be used as a binding element after providing them with a functional group. Also, DNA and RNA to which a functional group is introduced at their terminuses can be used as a binding element.

Substances having a nucleotide sequence complementary to a nucleotide sequence to be detected in a sample or a partial sequence thereof have 5 to 500 bases, preferably 10 to 300 bases, and more preferably 15 to 200 bases. Usually, they are DNA, RNA or PNA having a nucleotide sequence complementary to a nucleotide sequence to be detected.

When the binding element is a substance having a nucleotide sequence, the nucleotide sequence of the substance can be designed on the basis of known sequences. Examples of the substances having a nucleotide sequence include single-strand DNA chains, single-strand RNA chains, and single-strand PNA chains. As the nucleotide sequences possessed by the substances to be detected according to the present invention, among known sequences, those of genes that are known to undergo point mutation are also applicable. In this case, the region of the base in the binding element which is complementary to the base undergoing the mutation is preferably inside both ends of the binding element by more than three bases, and most preferably in the central region.

The binding element having a nucleotide sequence complementary to the nucleotide sequence possessed by the substance to be detected in a sample or a partial sequence thereof can be synthesized, for example, when the binding element is DNA, by using a DNA synthesizer in which chemical reactions of DNA synthesis are automated by the solid phase method using a carrier, such as silica. As the substrate of reaction, a nucleotide derivative in which the amino group of the base and the 5'-OH of ribose are protected by protecting groups and the 3'-OH of ribose is bound to diisopropyl phosphoamidite is used. As a protecting group for the amino group of the base, a benzoyl group, isobutyl group,etc., is mentioned, and as a protecting group for the 5'-OH of ribose, a dimethoxytrityl group, etc., is mentioned. In synthesizing DNA, a column in which any one of nucleotides, adenine, thymidine (T), cytosine, and guanine whose amino group and 5'-OH group are protected according to the nucleotide sequence is immobilized on a supporting material through the 3'-OH is used as a starting material, and after the removal of the trityl group by an acid (formation of 5'-OH), addition of the above-described nucleotide derivative having a phosphoamidite group at the 3'-terminus is carried out in the presence of an appropriate condensing agent such as tetrazole. The bond between them is then converted to a stable phosphoric triester bond using iodine and water. The cycle of detritylation and condensation reaction is repeated, and finally removal of the protecting groups and severance from the supporting material are carried out by treatment with ammonia. Through these steps, DNA as a target binding element can be synthesized. In case the spacer is single-strand DNA, it is possible to prepare such DNA by further synthesizing the sequence successively and the length is preferably more than 3 bases. In this manner, binding element DNA having a DNA spacer can be synthesized.

Now, the method of preparing a carrier to which a binding element is bound through or not through a spacer and which has an identifiable signal A is explained below.

To bind a protein as a binding element to the surface of a carrier, it is preferable that such a group that enables chemical binding by reaction, for example, a side chain or a terminal carboxyl group, amino group, glycidyl group, thiol group, hydroxyl group, amide group, imino group, hydroxysuccinyl ester group, maleimide group, etc. is present on the surface of the carrier. In the absence of such a group, it is possible to expose these groups that are capable of reaction by a physical treatment such as UV, radioactive rays, etc. or a chemical treatment. It is also possible to produce a functional group by coating another polymer over the surface.

Binding of a protein, etc. to the group capable of reaction which is present on the carrier can be carried out according to any of generally known chemical methods, for example, a method involving condensation with carbodiimide or a method of converting a carboxyl group into active ester to react with an amino group, which are known as methods to bind an amino group or a carboxyl group of a protein to a functional group on a carrier, a method in which thiol or maleimide is previously attached to a carrier and reacted with a maleimide group or a thiol group of a binding element, or a method in which an isothiocyanate group, a glycidyl group, or an N-hydroxysuccinimide group is previously attached to a carrier and reacted with an amino group of a binding element. The carrier prepared in this manner may be subjected to blocking by treating with BSA, etc. to suppress non-specific adsorptions or reactions.

When the spacer is polyethylene glycol diglycidyl, the binding element is a protein, and the carrier has epoxy groups on the surface, binding of the protein to the carrier can be carried out, for example, in the following manner. Ammonium hydroxide or hexamethylenediamine hydrochloride is added to the carrier particles in an amount of 10 to 100 times the molar quantity of the epoxy groups and reacted at 60-70°C at pH 10-12 for 0.5-2 days to aminate the surface of the particles. After the reaction, unreacted ammonium hydroxide or hexamethylenediamine hydrochloride is removed by washing by centrifugation and if necessary by carrying out dialysis using ion exchange water. To the carrier to which amino groups are introduced is added polyethylene glycol diglycidyl in an amount of 50 to 200 times the molar quantity of the amino groups and reacted at pH 10-12 at 20-40°C for 0.5-2 days to bind the spacer to the carrier. Binding of the epoxy groups possessed by the spacer to the protein is conducted according to the method described above.

As carriers used for binding DNA, etc. on the surface thereof, those having such a group that enables chemical binding by reaction, for example, a side chain or a terminal carboxyl group, amino group, glycidyl group, thiol group, hydroxyl group, amide group, imino group, hydroxysuccinyl ester group, and maleimide group on their surface can be used as they are. Carriers having no such group can also be used by exposing these groups that are capable of reaction through a physical treatment such as UV, radioactive rays, etc. or a chemical treatment. It is also possible to produce a functional group by coating another polymer over the surface.

Binding of DNA, etc. to the group capable of reaction which is present on the carrier can be carried out according to any of generally known chemical methods, for example, a method involving condensation with carbodiimide or a method of converting a carboxyl group into active ester to react with an amino group, which are known as methods to bind DNA to an amino group or a carboxyl group present on the surface of a carrier, a method in which thiol or maleimide is previously attached to a carrier and reacted with DNA having a maleimide group or a thiol group, or a method in which an isothiocyanate group, a glycidyl group, or an N-hydroxysuccinimide group is previously attached to a carrier and reacted with an amino group. It is also possible to synthesize DNA by successively polymerizing the bases of nucleic acid on the surface of the carrier using a method known as a method of DNA synthesis.

Methods to be used for the binding of a carrier, a spacer, and a binding element can be determined depending on the kinds of spacers, carriers, and binding elements. For example, when the binding element is DNA, it is possible to bind a carrier to the binding element generally by binding the amino group possessed by the base at the terminus of the binding element DNA to an epoxy group, a carboxyl group, an aldehyde group, a hydroxyl group, etc. on the carrier or on the spacer.

When each of the spacer and the binding element is single-strand DNA, and the carrier has an epoxy group on the surface, binding of the binding element DNA and the carrier is carried out, for example, according to the following procedures.

Binding element DNA having DNA as a spacer and single-strand DNA having a sequence complementary to the sequence of the binding element but not having the sequence of the spacer are synthesized in accordance with the method of DNA synthesis described above. They are then hybridized and formed into double-strand, the amino group in the base having the nucleotide sequence to be detected is protected and the amino group in the base of the liberated spacer region (if necessary an amino group is introduced to the terminus) is bound to the epoxy group on the carrier. After binding to the carrier, single-strand DNA having the sequence complementary to that of the binding element but not having the spacer sequence is allowed to dissociate, thereby the desired carrier-bound binding element DNA can be prepared.

A carrier prepared in this manner may be subjected to blocking by treating with BSA, etc. to suppress non-specific adsorptions or reactions.

Hybridization is carried out in an aqueous solution containing formamide, a salt, a protein, a stabilizer, a buffer, etc. as required. This solution is hereinafter referred to as "hybridization solution". The concentration of formamide is 0-60%. As salts, inorganic salts such as sodium chloride, potassium chloride, etc., organic acid salts such as sodium citrate, sodium oxalate, etc. may be mentioned, and the concentration of the salt is 0-2.0 mol/L, preferably 0.15-1.0 mol/L. As proteins, serum albumin, etc. may be mentioned, and as stabilizers, Ficoll, etc. may be mentioned. Furthermore, as buffers, phosphate buffer, etc. may be mentioned, and the concentration thereof is preferably 1-100 mmol/L. The above-mentioned hybridization of the two kinds of complementary DNAs can be achieved by adding each of the synthesized single-strand DNAs to the hybridization solution to have an equimolar concentration, warming at 50°-90°C for 1 minute to 6 hours, and by gradually cooling the reaction liquid.

In this manner, partially double-stranded DNA having a spacer of single-strand polynucleotide is prepared.

Binding of the prepared DNA to a carrier is carried out by washing carrier particles having epoxy groups with 1-100 mmol/L phosphate buffer to equilibrate the particles, mixing the carriers with the partially double-stranded DNA having a spacer of single-strand DNA prepared according to the above-described method in the same buffer as that used for the washing and keeping the mixture warm at 20°-50°C for 5-50 hours. After removing unreacted DNA by washing with an aqueous solution containing a salt such as 1-3 mol/L sodium chloride,etc., tris-hydrochloric acid buffer is added thereto, and the mixture is kept warm at 5 to 50 hours, thereby the unreacted epoxy groups on the carriers undergo ring-opening. Thus, double-strand DNA bound to the carriers is prepared.

By washing the carrier-bound double-strand DNA in the hybridization solution, single-strand DNA not having the spacer sequence is dissociated, and carrier-bound single-strand DNA can be prepared. Washing is carried out by repeating centrifugation at 1000-100,000g using the hybridization solution at 50°-90°C for several times.

When the spacer is polyethylene glycol diglycidyl, the binding element is a DNA chain, and the carrier has epoxy groups on the surface, binding of the binding element DNA and the carrier can be carried out in the same manner as in the above-described case where the spacer and the binding element are single-strand DNAs and the carrier has epoxy groups on the surface.

In addition to the chemical immobilization method using these synthetic polymers, a method is available in which streptavidin is immobilized on a carrier according to the method of Haun (Haun, M.) and Wasi (Wasi, S.) [Anal. Biochem., 191, 337 (1990)] and then a spacer containing biotin or a binding element having a biotin-labeled terminus itself can be immobilized on a carrier utilizing the affinity of streptavidin and biotin. Also available is a method in which two kinds of substances having an affinity biologically are respectively bound to a carrier and a spacer or to a carrier and a binding element itself, and then the respective two members are bound together utilizing the affinity of the members.

In binding a binding element to the above-described carrier provided with a signal A, a specific binding element S1 is bound to a carrier having A1 of a signal A. Similarly, another binding element S2 is bound to a carrier having A2 of a signal A. For example, a specific binding element S1 is bound to a carrier to which a bar code signal 01 is assigned and another specific binding element S2 is bound to a carrier to which a bar code signal 02 is assigned. According to the present invention, if a specific binding element and a signal A can be made to correspond to each other and thereby to become identifiable, there is no particular upper limit in the kinds of binding elements that can be detected in one reaction.

According to the present invention, as it is possible to have a specific nucleotide sequence S and a signal A correspond to each other and thereby become identifiable by using two or more of carriers in which binding elements each having a specific nucleotide sequence S1, S2 ... Sn are respectively bound to carriers each having A1, A2 ... An of a signal A through or not through a spacer, there is no particular upper limit in the kinds of nucleotides that can be detected in one reaction.

Next, method of preparing a substance to be detected is explained. Biological specimens used for the detection can be prepared from various organs, tissues, blood, etc. as such or using a method of extraction and purification of low molecular compounds, proteins, nucleic acid molecules such as DNA, RNA, etc.

The following will explain a method of preparing a sample having a nucleotide sequence to be detected.

When the sample to be used for the detection is, for example, DNA or RNA, the sample can be prepared from various organs, tissues, blood, etc. according to known methods for extracting DNA or RNA. The isolated DNA or RNA is cleaved with a specific restriction enzyme, etc. to be prepared into a single-strand DNA fragment or a single-strand RNA fragment each of 5-500 mer in length, preferably 10-300 mer in length containing a specific nucleotide sequence to be detected. It is also possible to use DNA, etc. in the sample, which is amplified by PCR, TMA or NASBA. According to the present invention, if the detection of a nucleotide sequence in a biological specimen is intended, it may be only necessary to fragment DNA, etc. In addition to biological specimens, compounds having a nucleotide sequence, which have been prepared by synthesis, etc. can also be used as samples.

Binding of a substance to be detected in a sample to a binding element is carried out in an aqueous solution which may contain a salt, a protein, a stabilizer, a buffer, a surfactant, etc. if necessary. As salts, inorganic salts such as sodium chloride, potassium chloride, etc., and organic acid salts such as sodium citrate, sodium oxalate, etc. may be mentioned and the concentration of a salt is 0-2.0 mol/L. As proteins, serum albumin, etc., may be mentioned. Furthermore, as buffers, phosphate buffer, etc. may be mentioned, and the concentration thereof is preferably 1-100 mmol/L. The binding is carried out with heating at 10°-70°C for 1-60 minutes and then by cooling if necessary.

The following will explain the method of mixing the carriers to which a binding element is bound through or not through a spacer and which have an identifiable signal A with a sample containing a substance to be detected in a solution, and binding the substance to be detected to the binding element on the carriers thereby to form a new signal B on the carriers.

When the binding element is a protein, formation of a signal B can be achieved in the following manner. A signal B may be any signal, so long as it is caused by a binding of a substance such as proteins, etc. to be detected to a substance such as proteins, etc. on the carriers (either one is a protein). While an increase in the weight may be directly measured utilizing the phenomena of resonance with oscillation produced by an oscillator, it is also possible to bind a labeling compound to an antibody, etc. that recognizes the substance to be detected and designate the label as a signal B. Otherwise, a labeling compound is bound to an antibody, etc. that recognizes the binding element in such manner that the antibody is unable to bind to the binding element when the substance to be detected is bound to the binding element, thereby the label of the unbound antibody can be detected as a signal B. Furthermore, in case two binding elements are linked together through a substance to be detected such as a protein, it is possible to detect a signal A possessed by one of the binding elements as a signal B.

Binding of a protein to be detected to a binding element is carried out usually in an aqueous solution under such conditions that do not cause denaturation of the protein, for example, in an aqueous solution having the buffer capacity and a pH of around 3-11 (the solution may contain a surfactant or an auxiliary agent to maintain the function of the protein) at 1°-90°C, preferably 5°-60°C.

If necessary, the labeling compound that has not been bound to the binding element and the residual substances in the sample are removed by separation through centrifugation or filtration.

As labeling compounds, fluorescent substances, light-emitting substances, enzymes, and compounds that emit a fluorescent light or a luminescent light by enzymes may be mentioned. Examples of fluorescent substances include fluorescein isothiocyanate (hereinafter abbreviated to FITC) and Texas Red.

For the light-emitting labeling, acridinium derivatives and ruthenium complex compounds may be used. For example, the acridiniums shown in US Patent Nos. 4,918,192, 4,946,958 and 4,950,613 or Clin. Chem. 29(8), 1474-1479 (1983) are preferable. Also, the ruthenium complex compound shown in Clin. Chem. 37(9), 1534-1539 (1991) is preferably used. This compound emits a light electrochemically together with an electron donor.

When a compound that emits a fluorescent light or a luminescent light by enzymes is the substrate, for example, luminol compounds, lucigenin compounds, etc. may be used as the substrate for peroxidase, and as the substrate for alkaline phosphatase, 3-(2'-adamantyl)-4-methoxy-4-(3"phosphoryloxy)phenyl-1,2-dioxetane salt, APS2, APS3 and APS5 [these three are luminescent light-emitting compounds produced by Lumigen Inc., H. Akhavan-Tafti, Z. Arghavani et al., John Wiley & Sons, Chichester 497-500 (1997)] which are phosphate derivatives of acridinium, etc. may be used.

When the labeling compound is a fluorescent substance, labeling can be carried out by binding carriers also to a fluorescent substance and utilizing the phenomenon that when a light is irradiated, one fluorescent substance which is bound emits a fluorescent light by energy transfer from the other fluorescent substance.

In detecting a signal B, in case a fluorescent light is to be detected, the area of the small hollow tube through which a solution containing carriers passes is placed at the detecting part of a fluorescence detector. Methods currently used for a cell sorter can be suitably used. Although no light source is necessary in the case of detecting an emitted light, it is preferable to provide a device to supply a light emission starter reagent or to add energy. As photodetectors, highly sensitive photocounters are suitable. In case the light emission is intense, the optical part of a fluorescence detector can also be used.

Now the following explains the method of forming a signal B on carriers which comprises mixing a sample containing the nucleotide sequence to be detected, in a solution, with carriers to which a binding element having a nucleotide sequence complementary to the nucleotide sequence to be detected or a partial sequence thereof is bound through or not through a spacer and which have an identifiable signal A, hybridizing the nucleotide sequence to be detected with that on the carriers having a complementary nucleotide sequence, thereby forming a new signal B on the carriers.

The signal B may be any signals that are caused by the double-strand formed by hybridization of the nucleotide sequence to be detected with that on carriers having a complementary nucleotide sequence. Although the absorbance of the double-strand may be directly measured, it is preferable to use a method of binding a compound having a tag capable of recognizing the hybridization to form the double strand. Examples of compounds having a tag capable of recognizing hybridization of the nucleotide sequence of the binding element to the nucleotide sequence of a sample include: compounds in which a labeling compound is bound to a nucleotide sequence complementary to the residual nucleotide sequence other than that complementary to the nucleotide sequence of the binding element possessed by a specific nucleotide sequence in a sample (hereinafter referred to as labeled binding element); compounds in which a labeling compound is bound to an antibody which recognizes hybridization of the nucleotide sequence of the binding element with that of a sample; and compounds in which a labeling compound is bound to an intercalator which recognizes hybridization of the nucleotide sequence of the binding element with that of a sample.

Hybridization is carried out in the hybridization solution described above under stringent conditions. For example, carriers bound to a binding element DNA and a sample containing a DNA fragment are added to the hybridization solution and heated at 50°-90°C, preferably 60°-75°C for 1 minute to 24 hours, preferably 5-15 minutes.

Next, the compound that has not hybridized to the binding element and has a nucleotide sequence complementary to that of the binding element in a sample, the compound which has not formed a complex with carriers having a signal A and in which a labeling compound is bound to a nucleotide sequence complementary to the residual nucleotide sequence other than that complementary to the nucleotide sequence of the binding element possessed by a specific nucleotide sequence in a sample, the compound in which a labeling compound is bound to an antibody which recognizes hybridization of the nucleotide sequence of the binding element with that of a sample, and the compound in which a labeling compound is bound to an intercalator which recognizes hybridization of the nucleotide sequence of the binding element with that of a sample are separated, if necessary by centrifugation or filtration.

Detection of the compound in which a labeling compound is bound to a labeled binding element in a sample, the compound in which a labeling compound is bound to an antibody which recognizes hybridization of the nucleotide sequence of the binding element with the nucleotide sequence in a sample, and the compound in which a labeling compound is bound to an intercalator which recognizes hybridization of the nucleotide sequence of the binding element with the nucleotide sequence in a sample can be carried out depending on the kinds of the tag attached to the compounds.

As examples of labeling compounds, the above-mentioned fluorescent substances, light-emitting substances, and compounds that emit a fluorescent light or a luminescent light by enzymes are mentioned.

Furthermore, since the hybridized region consists of a double-strand, it can be subjected to intercalation by adding, for example, an intercalator. In this case, compounds which emits fluorescent light by intercalation emit fluorescent light only in the presence of a hybridization product, therefore, a nucleotide sequence which does not have a complementary nucleotide sequence on the surface of carriers does not show fluorescence and thus it is not necessary to remove the residual intercalator and unhybridized sequences. In this sense, use of such compounds is particularly preferable.

As intercalators, compounds such as ethidium bromide, etc. and intercalators such as 4'.6-diamidino-2-phenylindole dihydrochloride n-hydrate (DAPI), SYBR Green 1(product of Molecular Probe), Pico Green (product of Molecular Probe), 2-methyl-4,6-bis-(4-N,N-dimethylaminophenyl)pyrylium (product of MBP Canon), 2-methyl-4,6-bis-(4-N,N-dimethylaminophenyl)thiopyrylium (product of Canon), etc. may be mentioned. Light emitting compounds such as acridinium ester, etc. that are decomposed, if not hybridized, under an alkaline condition and become incapable of emitting light are also suitably used.

When a labeled binding element is used, it is preferable that hybridization is carried out by adding labeled DNA having a sequence complementary to the sequence of the region close to the hybridized region and then the unhybridized labeled binding element is removed, thus providing a condition in which the labeled binding element is bound only to the hybridized one. Removal of unhybridized labeled binding element is preferably carried out by centrifugation or by a method using a filter. Instead of removing the unhybridized labeled binding element, unhybridized labeled binding element may be inactivated.

When the labeling compound is a fluorescent substance, labeling can be carried out by binding carriers also to a fluorescent substance and utilizing the phenomenon that when light is irradiated, one fluorescent substance which is hybridized emits fluorescent light by energy transfer from the other fluorescent substance. In this case, hybridization is carried out by adding a labeled product of DNA having a sequence complementary to that of a region closed to the hybridized region of DNA or RNA in the sample, to which a fluorescent material is bound. As energy is transferred from one fluorescent substance to the other when light is irradiated and only the hybridized emits fluorescent light, if a complementary DNA sequence or RNA sequence is absent on the carriers, the fluorescent light is weak or no fluorescent light is emitted at all, but if a complementary DNA sequence or RNA sequence is present on the carriers, it means that there is emitted a strong or at least some fluorescent light.

In detecting a signal B, in case fluorescent light is to be detected, the area of a small hollow tube through which a solution containing carriers passes is placed at the detecting part of a fluorescence detector. Methods currently used for a cell sorter can be suitably used. Although no light source is necessary in the case of detecting an emitted light, it is preferable to provide a device to supply a light emission starter reagent or to add energy. As photodetectors, highly sensitive photocounters are suitable. In case the light emission is intense, the optical part of a fluorescence detector can also be used.

It is preferable that the hollow tube consists of a thin tube and if cylindrical, has a shape such that carriers can pass in the longitudinal direction of the cylinder one by one. A solution supply means connecting to said hollow tube preferably has a hollow tube part which gently tapers into a small hollow tube. That is, it is preferable that carriers that have passed through the gently tapering tube of the solution supply means finally pass successively through the hollow tube that has been set to have a range of diameter such that two carriers are not able to pass it at one time. By gently tapering the tube, it is possible to prevent the tube from being clogged with carriers.

After the reaction, carriers hybridized or not hybridized with DNA or RNA are passed through the hollow tube. Since hybridized carriers emit a fluorescent light or a luminescent light or are in such a state that their fluorescence or luminescence intensity is strong against the background, at the time they pass, presence or absence of fluorescence or luminescence or their intensity is detected simultaneously with the detection of the signal of each carrier or at about the same time, the signals are serially taken in a computer, the signal of each carrier is related with the corresponding nucleotide sequence, and presence or absence of the target DNA or RNA sequence for detection is displayed. As a result, it is possible to detect the presence of the nucleotide sequence in the sample which corresponds to the signal shown by a carrier having a fluorescent or luminescent light (or strong fluorescence or luminescence). If it takes a long time for all the carriers to pass because the detecting part is small, two or more of signals and fluorescent light or luminescent light detecting parts are used and placed parallel to the flow channel, and thus information detected by each detecting part can be compiled in the data processing means for analysis.

When the solution is passed through a tube having a small hollow tube such that only one such carrier as above-mentioned can pass at one time, while a signal A possessed by the carrier and a signal B which is formed on the carrier when the nucleotide sequence to be detected is present in the sample can be detected almost simultaneously, signals A and B may be detected separately so long as it can be identified that both signals are thrown out from the same carrier. Where a digital signal is to be detected, currently, various apparatuses that accurately read digital signals have been developed and any such apparatus can be used if it is capable of reading delicate signals. Reading of a delicate digital signal can be carried out, for example, by optically enlarging the signal and reading it based on the principle used for the bar code touch scanner; by once taking the signal as an image using a CCD camera, etc. and reading it as such or after enlargement by a reading device; or by irradiating a delicate laser to the carriers and reading the signal using the reflected light or transmitted light. If recording is done by such magnetoptical means as used in CD, reading is preferably carried out optically by laser in the same manner as used in CD.

According to the present invention, it is also possible to quantitatively determine the concentration of a substance to be detected in a sample such as DNA by preliminarily preparing a calibration curve using a known amount of the substance to be detected such as a DNA sample.

The present invention enables the following applications.

By using a serum of a patient as a test sample, binding a suitable antigen of various infectious diseases to carriers, and using as a tag a preparation in which a fluorescent labeling compound or a light emitting labeling compound is bound to anti-human immunoglobulin, it is possible to identify in one operation which kind of infectious diseases the patient is infected with.

By using a serum of an allergic patient as a test sample, binding a suitable antigen of various allergens to carriers, and using as a tag a preparation in which a fluorescent labeling compound or a light emitting labeling compound is bound to anti-human immunoglobulin, it is possible to identify in one operation which kind of allergens the patient is sensitized.

When a lysate of a certain cell is used as a sample, various proteins or low molecular compounds are bound to carriers and a preparation in which a fluorescent labeling compound or a light emitting labeling compound is bound to an antibody against the various proteins or the low molecular compounds bound to the carriers is used as a tag, the labeled antibody can not react if a substance having an affinity to various proteins or low molecular compounds bound to the carriers is present in the sample, which means the presence in the sample of a substance having an affinity to the bound product which corresponds to carriers to which a fluorescence labeling compound or a fluorescent light emitting labeling compound is not bound, and thus a substance in the cell is detected in one operation. In this case, when DNA is bound to the carriers, it is also possible to detect the presence of a protein having an affinity to the DNA, for example, a protein binding to an expression promoter of the DNA. Furthermore, when a substance capable of binding proteins attached on the carriers to each other is present in the sample, the two carriers associate with each other with their sides on which binding element is present oppositely facing. Such a substance can also be detected by adjusting the direction of a bar code, etc. or intervals of detection when detection is carried out so that such an association can also be determined.

In recent years, with the progress of the gene cloning, and gene sequencing technologies, numbers of novel genes have been newly obtained. However, the functions possessed by many of the genes are yet to be clarified. The present invention is also applicable to elucidation of the functions of proteins encoded by such gene sequences whose functions are unclear. For example, such a gene fragment is allocated 5' upstream and a gene sequence encoding a green fluorescent protein (hereinafter referred to as "GFP") is linked 3' downstream thereto to express the protein encoded by the gene as a fused protein with GFP. After immobilizing the protein encoded by such fluorescent-labeled, function-unknown gene on carriers, the carriers are incubated with carriers which are provided with bar codes and on which various known substances are immobilized, and a substance binding to the protein encoded by the function-unknown gene is monitored using bar codes and fluorescent signals, thereby the substance binding to such substance can be screened for. That is to say, by using the method of the present invention, it becomes possible to readily analyze the function of a protein encoded by a gene sequence with unknown function. If GFPs having a mutation therein are utilized, since they each have a different fluorescence, a two or more of function-unknown genes can be analyzed efficiently at one time by binding GFPs each having a different fluorescence 3' downstream to the gene which is the object of analysis.

The labeling compounds used in the above example are not necessarily GFPs, and any compounds that are capable of expressing a protein from a gene by a genetic engineering technique and have certain signals can be utilized. For example, enzymes such as aequorin and luciferase may be used in the detection system.

A certain protein interacts with a protein binding thereto, low molecular compounds or a substance acting on proteins such as promoters, etc., and bind to each other in a solution under the conditions similar to in vivo conditions. For example, a complex of the protein and the substance interacting therewith is formed on the carriers by attaching the protein to carriers, adding a substance that interacts with the protein thereto, and incubating. In case the substance is capable of binding the proteins bound on the carriers together, the two carriers associate with each other with their side of binding oppositely facing. To identify the formed complex, a labeling compound that binds to the complex and identify the kind of the complex is further reacted therewith to detect the presence or absence of a tag. In this manner, it is possible to determine whether or not a certain protein and a substance that interacts therewith are present.

### Brief Description of the Drawings

Fig. 1 shows a schematic view of an apparatus of the present invention.
Fig. 2 shows a schematic view of an apparatus of the present invention.

### Best Modes for Carrying out the Invention

### Example 1

### (1) Preparation of carriers having a signal A

Polymethylmethacrylate fibers (product of Mitsubishi Rayon) each having a diameter of 1.5 mm and a length of 15 cm were incubated in 1 mol/L aqueous NaOH solution at 80°C for 2 hours, thereby the surface was hydrolyzed into Na salt of carboxyl groups. After washing, the Na salt was once restored to carboxyl groups with 0.02 mol/L hydrochloric acid solution. To the fibers were directly printed bar codes 01 of Code 128 using a TDK Laser Marker CLM-03 printer while rotating the fibers. The bar code-printed areas were cut out to prepare carriers having a length of 15 mm. Similarly, bar codes 02 were printed and cut out to prepare similar carriers.

### (2) Preparation of carrier-bound single-strand DNA (carrier-bound binding element)

Fifty carriers having a bar code 01 were dispersed into 10 ml of HEPES buffer, to which 1 µmol of oligo DNA having the nucleotide sequence shown in SEQ ID NO: 1 which consists of 15 bases having an amino group at the 5' terminus was added. WSC [water soluble carbodiimide, compound name: 1-ethyl-3-(-dimethylaminopropyl)carbodiimide, hydrochloride, product of Dojin Kagaku] (50 µmol) was added thereto, thereby the oligo DNA having the nucleotide sequence shown in SEQ ID NO:1 was immobilized on the carriers through amino groups. The carriers to which the oligo DNA having the nucleotide sequence shown in SEQ ID NO: 1 is bound were subjected to blocking in 1% BSA solution, and washed with distilled water, thereby carriers to which the oligo DNA having the nucleotide sequence shown in SEQ ID NO: 1 is bound and having a bar code 01 were prepared. The 5' terminus AAA in the nucleotide sequence shown in SEQ ID NO: 1 was inserted as a spacer.

Similarly, oligo DNA having the nucleotide sequence shown in SEQ ID NO: 2 which consists of 15 bases having an amino group at the 5'-terminus was bound to 50 carriers having a bar code 02. After similarly treating the carriers, carriers to which oligo DNA having the nucleotide sequence shown in SEQ ID NO: 2 is bound and having a bar code 02 were prepared.

### (3) Preparation of fluorescent-bound single-strand DNA (fluorescente-bound binding element)

A solution of 2 nmol/ml fluorescent-labeled oligo DNA prepared by reacting FITC (fluorescein isothiocyanate) with the 3'-terminus of oligo DNA having the nucleotide sequence shown in SEQ ID NO: 3 was prepared.

### (4) Preparation of a DNA test sample

Oligo DNA having the nucleotide sequence shown in SEQ ID NO: 4 which has a sequence complimentary to the nucleotide sequence shown in SEQ ID NO: 1 and that shown in SEQ ID NO: 3 was prepared and dissolved in TE buffer (10 mmol/L Tris and 1 mmol/L EDTA) at a concentration of 1 nmol/ml to prepare a test sample solution.

Also prepared was oligo DNA having the nucleotide sequence shown in SEQ ID NO: 5 which has a sequence not complementary to the nucleotide sequence shown in SEQ ID NO: 1 and that shown in SEQ ID NO: 3, which was dissolved in TE buffer at a concentration of 1 nmol/ml to prepare a test sample.

### (5) Hybridization

To 10 ml of 0.05 mol/L HEPES buffer (pH 7, containing 1 mol/L NaCl) containing each 5 carriers having a 01 or 02 bar code prepared in (2) above was added 100 µL of the fluorescent-labeled single-strand DNA (fluorescent-bound binding element) prepared in (3) above. Then, 100 µL of the DNA test sample having the nucleotide sequence shown in SEQ ID NO: 4 or DNA test sample having the nucleotide sequence shown in SEQ ID NO: 5 both prepared in (4) above, or TE buffer was added thereto, and the respective mixtures were warmed at 50°C for 30 minutes while slowly stirring.

The resulting mixtures were respectively filtered through a 0.8 µm cellulose acetate membrane filter (C080A047A) (product of Toyo Roshi) and washed with 0.05 mol/L HEPES buffer (pH 7, containing 1 mol/L NaCl) and the carriers were again dispersed in 10 ml of the same buffer.

### (6) Detection

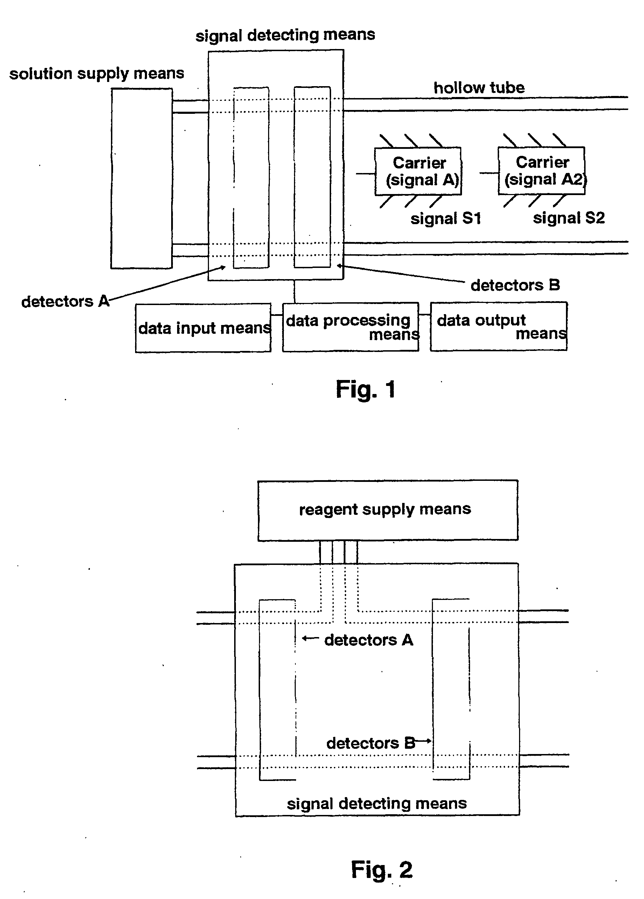

An apparatus shown in Fig. 1 was prepared, and the bar code and fluorescence were detected while passing the respective solutions prepared in (5) above through a hollow tube in the signal detection means. As the bar code reader, a hand laser scanner SL-114 (product of Nichiei Intec) was used. As a fluorescence detector, a Hitachi F-4000 spectrophotofluorometer was used, and fluorescence intensity was measured at an excited light wavelength of 495 nm and a fluorescent light wavelength of 520 nm. The hollow tube consisted of a quartz glass tube having an inner diameter of 2 mm, and the flow rate was 0.6 cm/sec. Because there was a time-lag of 36 seconds between the detection of the bar code signal and that of the fluorescent signal, the time-lag was used to detect the signal which corresponds to the fluorescent signal on the carrier having a specific bar code.

Maximum value of the fluorescence intensities being monitored while taking noise components into consideration was defined as the fluorescence intensity, and the mean value of the fluorescence intensities of 5 carriers having a bar code 02 and having the same nucleotide sequence was defined as 1.

The results are shown in Table 1.

**Table 1**

| Bar code Signal | No DNA | DNA having the nucleotide sequence shown in SEQ ID NO: 4 | DNA having the nucleotide sequence shown in SEQ ID NO: 5 |
|---|---|---|---|
| 01 | 1.3 | 53.8 | 1.7 |
| 02 | 1.0 | 2.5 | 0.9 |

As a result, the fluorescence value corresponding to the carriers with a bar code 01 was observed to be remarkably high. In the cases of the DNA test sample having the nucleotide sequence shown in SEQ ID NO: 5 which is not complementary to the nucleotide sequence of the binding element DNA of a bar code 01 and the sample only containing carriers (no DNA), the fluorescence values corresponding to the carriers having either of a bar code signals were low, and it was demonstrated that the method of the present invention enabled specific detection of a specific sequence.

### Example 2

To 10 ml of 0.05 mol/L HEPES buffer (pH 7, containing 1 mol/L NaCl) containing each 5 carriers having a 01 or 02 bar code prepared in Example 1 (2) was added 100 µL of the DNA test sample having the nucleotide sequence shown in SEQ ID NO: 4 or the DNA test sample having the nucleotide sequence shown in SEQ ID NO: 5 both prepared in Example 1 (4), or TE buffer, and the respective mixture were warmed at 50°C for 30 minutes while slowly stirring. Then, 10 ml of a solution prepared by diluting SYBR Green I solution 5000-fold with TE buffer was added thereto, and after warming the mixture at room temperature for 30 minutes while slowly stirring, measurement was carried out using an apparatus shown in Fig. 1 in the same manner as in Example 1. Fluorescence intensity was measured at an excited light wavelength of 254 nm and a fluorescent light wavelength of 520 nm.

As in Example 1, the fluorescence intensities were shown with the mean value of the fluorescence intensities of 5 carriers having a bar code 02 and having the same nucleotide sequence being defined as 1.

The results are shown in Table 2.

**Table 2**

| Bar code Signal | No DNA | DNA having the nucleotide sequence shown in SEQ ID NO: 4 | DNA having the nucleotide sequence shown in SEQ ID NO: 5 |
|---|---|---|---|
| 01 | 2.3 | 38.6 | 3.1 |
| 02 | 1.0 | 1.5 | 2.4 |

As a result, the fluorescence value corresponding to the carriers with a bar code 01 was observed to be remarkably high. In the cases of the DNA test sample having the nucleotide sequence shown in SEQ ID NO: 5 which can not complementary to the nucleotide sequence of the binding element DNA of a bar code 01 and the sample only containing carriers (no DNA), the fluorescence values corresponding to the carriers having either of the bar code signals were low, and it was demonstrated that the method of the present invention enabled specific detection of a specific sequence. It was also demonstrated that according to the present invention, after the hybridization operation, measurement could be simply carried out without requiring separation of the carriers and the unbound tag.

### Example 3

Acridinium-I (product of Dojin Kagaku) was bound to oligo DNA in which an amino group is introduced to the 6th guanine from the 5' terminus of oligo DNA having the nucleotide sequence shown in SEQ ID NO: 3 to prepare 2 nmol/ml solution thereof (hereinafter referred to as acridinium-labeled binding element).

To 500 µL of a solution comprising 0.1 mol/L lithium succinate buffer (pH 5), 20% lithium lauryl sulfate, 0.4 mol/L LiCl, 20 mmol/L EGTA and 20 mmol/L EDTA and containing each 5 carriers having a bar code 01 or 02 prepared in Example 1 (2), was added 10 µL of the DNA test sample having the nucleotide sequence shown in SEQ ID NO: 4 or the DNA test sample having the nucleotide sequence shown in SEQ ID NO: 5 each prepared in Example 1 (4) or TE solution. The above-mentioned acridinium-labeled binding element was further added thereto to have a concentration of 0.1 pmol/L, and hybridization reaction was carried out by warming the respective mixtures at 60°C for 30 minutes while slowly stirring. Then, 1500 µL of 0.6 mol/L Tetraborate buffer (pH 8.5), 5% tritonX-100 and TETRA solution were added thereto, the mixture was warmed at 60°C for 20 minutes to hydrolyze the unreacted acridinium-labeled binding element, and the reaction vessels containing the samples were immersed in water for rapid cooling.

Detection was carried out using an apparatus in which the signal detecting means in Fig. 1 is replaced by that of Fig. 2. After the reaction, the sample was led to a tube, and the signals were detected with a bar code reader. Then, 2 mol/L NaOH solution containing 0.03% H₂O₂ was introduced through the reagent supply means and mixed with the sample within the tube. Immediately, the light emission count was measured using the detecting part of a Lumicounter 2500 luminescent detector (product of Microtech Nichion), and a clear chemical luminescence count could be detected only with respect to the sample comprising carriers to which a bar code 01 is attached and with which DNA having the nucleotide sequence shown in SEQ ID NO: 4 was reacted.

### Example 4

### (1) Preparation of carriers having a signal A

Carriers having a signal A were prepared in the same manner as in Example 1 (1).

### (2) Preparation of a carrier-bound protein (carrier-bound antibody)

Fifty carriers having a bar code 01 were dispersed in 10 ml of HEPES buffer to which 100 µmol of hydroxysuccinimide (product of Kanto Kagaku) was added, followed by the addition of 100 µmol of WSC (water soluble carbodiimide, compound name: 1-ethyl-3-(-dimethylaminopropyl)carbodiimide, hydrochloride, product of Dojin Kagaku) to succinimidate the carboxyl groups. The carriers were washed with the same buffer, and 10 ml of a buffer containing 1 µmol/ml anti-human immunoglobulin γ-chain antibody was immediately added thereto to immobilize the antibody. Furthermore, blocking was carried out in 1% BSA solution, followed by washing with distilled water to prepare carriers to which anti-human immunoglobulin γ-chain antibody is bound and which have a bar code 01.

Similarly, anti-human immunoglobulin µ-chain antibody was bound to 50 carriers having a bar code 02 and similar procedures were followed to prepare carriers to which anti-human immunoglobulin µ-chain antibody is bound and which have a bar code 02.

### (3) Preparation of fluorescent-bound antibody

Solution A (2 nmol/ml) in which human IgG was fluorescent-labeled by reacting with FITC (fluorescein isothiocyanate) was prepared. Similarly, solutions (2 nmol/ml each) in which human IgA and IgM were respectively fluorescent-labeled by reacting with FITC (fluorescein isothiocyanate) were prepared (respectively, solution B and solution C).

### (4) Reaction of a protein

To 10 ml of 0.05 mol/L HEPES buffer (pH 7) containing each 5 carriers having a bar code 01 or a bar code 02 prepared in (2) above were added 100 µL of the fluorescent-bound antigen solution A and 100 µL of the fluorescent-bound solution B and warmed at 37°C for 30 minutes while slowly stirring.

The mixtures were respectively filtered through a 0.8 µm cellulose acetate membrane filter (product of Toyo Roshi, C080A047A), and washed twice with 0.05 mol/L HEPES buffer (pH 7) and the carriers were again dispersed in 10 ml of the same buffer, which was designated as detection test sample solution X.

Separately, 100 µL of the fluorescent-bound antigen solution C and 100 µL of the fluorescent-bound solution B were added to 10 ml of 0.05 mol/L HEPES buffer containing each 5 carriers having a bar code 01 or 02 prepared in (2) above, and the respective mixtures were warmed at 37°C for 30 minutes while slowly stirring.

The mixtures were respectively filtered through a 0.8 µm membrane filter (product of Toyo Roshi, C080A047A), and washed twice with 0.05 mol/L HEPES buffer (pH 7) and the carriers were again dispersed in 10 ml of the same buffer, which was designated as detection sample Y.

### (5) Detection

An apparatus shown in Figs. 1 and 2 was prepared and the bar code and fluorescence were detected while passing the solutions of detection samples X and Y prepared in (4) above through a hollow tube in the detection device. As the bar code reader, a hand laser scanner SL-114 (product of Nichiei Intec) was used. As a fluorescent detector, a Hitachi F-4000 spectrophotofluorometer was used, and measurement was carried out at an excited light wavelength of 495 nm and a fluorescent light wavelength of 520 nm. The hollow tube consisted of a quartz glass tube having an inner diameter of 2 mm and the flow rate was 0.6 cm/sec. Because there was a time-lag of 36 seconds between the detection of the bar code signal and that of the fluorescent signal, the time-lag was used to detect the signal which corresponds to the fluorescent signal on the carrier having a specific bar code.

The results are shown in Table 3.

**Table 3**

| Bar code signal | No test sample | Test sample X | Test sample Y |
|---|---|---|---|
| 01 | 2.4 | 87.0 | 2.6 |
| 02 | 3.0 | 3.9 | 71.3 |

Maximum value of the fluorescence intensities being monitored while taking noise components into consideration was defined as the fluorescence intensity, and the mean value of the fluorescence intensities of 5 carriers having a bar code 02 was defined as 1.

As a result, in the case of the test sample X, the fluorescence value corresponding to the carriers having a bar code 01 was observed to be remarkably high. That is, in the test sample X which contains human IgG that reacts with anti-human IgG immobilized on the bar code 01 carriers, the fluorescent light corresponding to the bar code 01 carriers was strongly detected, whereas, in the case of the test sample Y, the fluorescence value corresponding to the bar code 02 carriers was observed to be remarkably high. In other words, in the test sample Y which contains human IgM that reacts with anti-human IgM immobilized on the bar code 02 carriers, the fluorescent light corresponding to the bar code 02 carriers was strongly detected. Since the fluorescence values were not enhanced in the "no test sample" case, the method of the present invention enabled specific detection of a protein (antigen) that reacts with a specific protein (antibody).

### Example 5

### (1) Preparation of avidin-bound carriers and mouse immunoglobulin-bound carriers respectively having a bar code tag

Carriers of 15 mm in length having a bar code 01 or 02 were prepared by using polymethylmethacrylate fibers as a raw material and introducing carboxyl groups on the surface thereof in the same manner as in Example 1. As in Example 1, 50 carriers having a bar code 01 were dispersed in 10 ml HEPES buffer, 100 µmol hydroxysuccinimide was added thereto, followed by the addition of 100 µmol WSC to succinimidate carboxyl groups, and after washing the carriers with the same buffer, 10 ml of a buffer containing 1 µmol/ml avidin was immediately added thereto to immobilize avidin on the carriers. The carriers were subjected to blocking in 1% BSA solution, and washed with distilled water to prepare carriers to which avidin is bound and which have a bar code 01.

Similarly, carriers to which mouse immunoglubulin G is bound and which have a bar code 02 were prepared by binding mouse immunoglubulin G to 50 carriers which have a bar code 02 and treating in the same manner.

### (2) Preparation of polypeptide test sample solution

An FITC-labeled polypeptide having the nucleotide sequence shown in SEQ ID NO: 6 which consists of 15 amino acids containing avidin-binding peptide sequence and having FITC at the amino-terminus was synthesized and dissolved in PBS containing 0.1% BSA at a concentration of 2 nmol/ml to prepare a test sample solution.

Similarly, a FITC-labeled polypeptide having the nucleotide sequence shown in SEQ ID NO: 7 which consists of 15 amino acids without containing avidin-binding peptide sequence and having FITC at the amino-terminus was synthesized and dissolved in PBS containing 0.1% BSA at a concentration of 2 nmol/ml to prepare a test sample solution.

### (3) Reaction of polypeptide test sample solutions with carriers

To 10 ml of 0.05 mol/L HEPES buffer (pH 7) containing each 5 carriers having a bar code 01 or a bar code 02 prepared in (1) above was added each 100 µL of the test sample having the amino acid sequence shown in SEQ ID No: 6 or the test sample having the amino acid sequence shown in SEQ ID NO: 7, both prepared in (2) above, or 0.1% BSA/PBS without containing polypeptide, and the respective mixtures were warmed at 37°C for 30 minutes while slowly stirring.

The mixtures were then respectively filtered through a 0.8 µm cellulose acetate membrane filter (product of Toyo Roshi, C080A047A), and washed twice with 0.05 mol/L HEPES buffer (pH 7), and the carriers were again dispersed in 10 ml of the same buffer.

### (4) Detection

An apparatus shown in Figs. 1 and 2 was prepared, and the bar code and fluorescence were detected while passing the solutions prepared in (3) above through a hollow tube in the detection device. As the bar code reader, a hand laser scanner SL-114 (product of Nichiei Intec) was used. As a fluorescent detector, a Hitachi F-4000 spectrophotofluorometer was used, and measurement was carried out at an excited light wavelength of 495 nm and a fluorescent light wavelength of 520 nm. The hollow tube consisted of a quartz glass tube having an inner diameter of 2 mm and the flow rate was 0.6 cm/sec. Because there was a time-lag of 36 seconds between the detection of the bar code signal and that of the fluorescent signal, the time-lag was used to detect the signal which corresponds to the fluorescent signal on the carrier having a specific bar code.

The results are shown in Table 4.

**Table 4**

| Bar code Signal | No DNA | Sample having the amino acid sequence shown in SEQ ID NO: 6 | Sample having the amino acid sequence shown in SEQ ID NO: 7 |
|---|---|---|---|
| 01 | 0.9 | 74.2 | 1.2 |
| 02 | 1.0 | 1.3 | 1.1 |

Maximum value of the fluorescence intensities being monitored while taking noise components into consideration was defined as the fluorescence intensity, and the mean value of the fluorescence intensities of 5 carriers having a bar code 02 in the absence of any test sample was defined as 1.

As a result, in the case of the test sample having the nucleotide sequence shown in SEQ ID NO: 1, the fluorescence value corresponding to the carriers having a bar code 01 was observed to be remarkably high. That is, in the test sample of the nucleotide sequence shown in SEQ ID NO: 1 containing a peptide sequence that reacts with avidin of a bar code 01. the fluorescent light corresponding to the bar code 01 carriers was strongly detected, whereas, in the cases of the test sample having the nucleotide sequence shown in SEQ ID NO: 2 and the solution containing no test sample, the fluorescence values corresponding to the carriers having either bar code signal were not enhanced, and the method of the present invention enabled specific detection of a polypeptide sequence that reacts with a specific protein (avidin).

### Industrial Applicability

According to the present invention, a method of detecting a specific nucleotide sequence with wide applicability at low cost, a reagent for detecting a specific nucleotide sequence, and an apparatus for detecting a specific nucleotide sequence are provided.

[Sequence Listing Free Text]
SEQ ID NO: 1 - Description of the artificial sequence:
synthetic DNA
SEQ ID NO: 2 - Description of the artificial sequence:
synthetic DNA
SEQ ID NO: 3 - Description of the artificial sequence:
synthetic DNA
SEQ ID NO: 4 - Description of the artificial sequence:
synthetic DNA
SEQ ID NO: 5 - Description of the artificial sequence:
synthetic DNA
SEQ ID NO: 6 - Description of the artificial sequence:
synthetic peptide
SEQ ID NO: 7 - Description of the artificial sequence:
synthetic peptide

## Claims

1. A method of detecting a substance to be detected in a sample **characterized by** mixing a sample containing the substance to be detected, in a solution, with carriers which have a shape such that, when suspended in a solution and flowed through a hollow tube for detecting signals formed in a signal detecting means capable of detecting a signal A and a signal B, two or more of the carriers do not move in an overlapping manner to the cross section of the hollow tube, to which a substance capable of binding to the substance to be detected (hereinafter referred simply to as "binding element") is bound through or not through a spacer, and which have an identifiable signal A; binding the substance to be detected to said binding element thereby causing a signal B to be formed on the carriers; flowing a suspension of said carriers through the hollow tube by a solution supply means connected to the hollow tube; detecting the signal A and the signal B by the signal detecting means; and relating the signal A with the signal B.

2. A method according to claim 1, wherein said substance to be detected is a substance having a specific nucleotide sequence and said binding element is a substance having a nucleotide sequence complimentary to the nucleotide sequence of the substance to be detected or a partial sequence thereof.

3. A method according to claim 2, wherein the binding of the substance to be detected to the binding element is carried out by hybridization of the complementary nucleotide sequences.

4. A method according to claim 3, wherein the identifiable signal A is related with the nucleotide sequence of the binding element.

5. A method according to claim 3, wherein the carriers have a mutually identifiable signal A which is related with the nucleotide sequence of the binding element and are in the form of a mixture of two or more of carriers having a mutually different signal A.

6. A method according to any of claims 2 to 5, wherein the binding element is DNA, RNA or PNA having 5 to 500 bases.

7. A method according to any of claims 3 to 6, wherein a signal B is formed on the carriers by binding a compound having a tag capable of recognizing hybridization between the nucleotide sequence of the binding element and the nucleotide sequence of the substance to be detected.

8. A method according to claim 7, wherein said compound having a tag capable of recognizing the hybridization is a compound in which a compound having a nucleotide sequence complementary to the residual nucleotide sequence other than the sequence complementary to the nucleotide sequence of the binding element possessed by the substance to be detected is bound to a labeling compound.

9. A method according to claim 8, wherein said compound having a nucleotide sequence complementary to the residual nucleotide sequence other than the sequence complementary to the nucleotide sequence of the binding element possessed by the substance to be detected is DNA, RNA or PNA having 5 to 500 bases.

10. A method according to claim 7, wherein said compound having a tag capable of recognizing the hybridization is a compound in which an antibody recognizing the hybridization is bound to a labeling compound.

11. A method according to claim 7, wherein said compound having a tag capable of recognizing the hybridization is a compound in which an intercalator recognizing the hybridization is bound to a labeling compound.

12. A method according to claim 11 which further includes a step of separating the compound having a tag which has not been bound to the carriers from the carriers.

13. A method according to any of claims 7 to 12, wherein the labeling compound is a fluorescent substance, a light emitting substance, an enzyme or a compound which emits a fluorescent light or a luminescent light by means of an enzyme.

14. A method according to claim 13, wherein the carriers are bound to a substance emitting a fluorescent light and the labeling compound is a compound which emits a fluorescent light by energy transfer.

15. A method according to claim 7, wherein said compound having a tag capable of recognizing the hybridization is a compound which forms a signal B by intercalation.

16. A method according to claim 1, wherein said binding element is a protein capable of binding to a substance to be detected.

17. A method according to any of claims 1 to 16, wherein the identifiable signal A is a digital signal.

18. A method according to claim 17, wherein the digital signal A is magnetic or optical.

19. A method according to claim 17, wherein the digital signal A is a bar code.

20. A method according to any of claims 1 to 19, wherein the shape of the hollow tube is cylindrical and the carrier is cylindrically shaped with its outer shape smaller than the inner diameter of the hollow tube, the length of said cylindrical shape being larger than the inner diameter of the hollow tube.

21. A method according to any of claims 1 to 20, wherein the carrier is cylindrical or spherical.

22. A method according to claim 21, wherein the carrier internally contains magnetic particles.

23. A method according to any of claims 1 to 22, wherein the carrier is cylindrical and has an outer diameter of 1 µm to 2 mm and a length of 10 µm to 2 cm.

24. A method according to any of claims 1 to 23, wherein the carrier has a specific gravity of 0.8 to 2.0.

25. A method according to any of claims 1 to 24, wherein the surface of the carrier is coated with a resin.

26. A carrier to which a binding element capable of binding to a substance to be detected is bound through or not through a spacer and which has an identifiable signal A.

27. A carrier according to claim 26, wherein said binding element has a nucleotide sequence complementary to the nucleotide sequence of a substance to be detected or a partial sequence thereof.

28. A carrier according to claim 26, wherein said binding element is a protein capable of binding to a substance to be detected.

29. A carrier according to any of claims 26 to 28, which internally contains magnetic particles.

30. A carrier according to any of claims 26 to 29, wherein the signal A is a digital signal.

31. A carrier according to claim 30, wherein said digital signal is a bar code.

32. A carrier according to any of claims 26 to 31, which is cylindrical or spherical.

33. A carrier according to any of claims 26 to 32, which is cylindrical and has an outer diameter of 1 µm to 2 mm and a length of 10 µm to 2 cm.

34. A carrier according to any of claims 26 to 33 having a specific gravity of 0.8 to 2.0.

35. A carrier according to any of claims 26 to 34 having a surface coated with a resin.

36. An apparatus to detect a specific substance, **characterized by** having a signal detecting means, a data input means, a data processing means, and a data output means: said signal detecting means being provided with signal detectors A and B capable of detecting at least two kinds of signals A and B present on a carrier to which a binding element having a nucleotide sequence complementary to a nucleotide sequence to be detected or a partial sequence thereof is bound through or not through a spacer, said signal detectors A and B having a hollow tube through which said carrier is flowed, and said carrier having a structure such that the carriers do not move in an overlapping manner to the cross section of said hollow tube; and said data processing means being provided with a function to relate a signal A on the carrier with a specific substance to be detected using data and signals A and B, said data specifying said specific substance that has been related with the signal A on the carrier and being input from the data input means, and said signals A and B having been detected by the signal detecting means, and to output said related data to the data output means.
